(19) Europäisches Patentamt
European Patent Office
Office européen des brevets

(11) **EP 2 456 756 B1**

(12) **EUROPÄISCHE PATENTSCHRIFT**

(45) Veröffentlichungstag und Bekanntmachung des Hinweises auf die Patenterteilung:
**14.06.2017 Patentblatt 2017/24**

(21) Anmeldenummer: **10734978.9**

(22) Anmeldetag: **16.07.2010**

(51) Int Cl.:
*C07C 319/02* (2006.01)    *C07C 319/28* (2006.01)
*C07C 321/04* (2006.01)    *C01B 17/16* (2006.01)

(86) Internationale Anmeldenummer:
**PCT/EP2010/060322**

(87) Internationale Veröffentlichungsnummer:
**WO 2011/009817 (27.01.2011 Gazette 2011/04)**

(54) **VERFAHREN ZUR KONTINUIERLICHEN HERSTELLUNG VON METHYLMERCAPTAN AUS KOHLENSTOFFHALTIGEN VERBINDUNGEN, SCHWEFEL UND WASSERSTOFF**

METHOD FOR THE CONTINUOUS PRODUCTION OF METHYL MERCAPTAN FROM CARBON-CONTAINING COMPOUNDS, SULFUR, AND HYDROGEN

PROCÉDÉ DE PRODUCTION EN CONTINU DE MERCAPTAN MÉTHYLIQUE À PARTIR DE COMPOSÉS CARBONÉS, DE SOUFRE ET D'HYDROGÈNE

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**

(30) Priorität: **20.07.2009 DE 102009027837**

(43) Veröffentlichungstag der Anmeldung:
**30.05.2012 Patentblatt 2012/22**

(73) Patentinhaber: **Evonik Degussa GmbH 45128 Essen (DE)**

(72) Erfinder:
• BARTH, Jan-Olaf
  40591 Düsseldorf (DE)
• WECKBECKER, Christoph
  63584 Gründau-Lieblos (DE)
• REDLINGSHÖFER, Hubert
  91481 Münchsteinach (DE)
• LERCHER, Johannes A.
  85521 Ottobrunn (DE)
• KAUFMANN, Christoph
  83527 Haag (DE)

(56) Entgegenhaltungen:
WO-A2-2009/083368    US-A- 2 565 195
US-A- 3 880 933    US-A- 4 410 731

• MASHKINA A V ET AL: "ACTIVITY OF TUNGSTATE CATALYSTS IN THE SYNTHESIS OF METHYL - MERCAPTANE FROM METHANOL AND HYDROGEN SULFIDE" REACTION KINETICS AND CATALYSIS LETTERS, ELSEVIER, AMSTERDAM, NL LNKD-DOI:10.1007/BF02071157, Bd. 36, Nr. 1, 1. Januar 1988 (1988-01-01) , Seiten 159-164, XP002063476 ISSN: 0133-1736
• CHEN A ET AL: "Direct synthesis of methanethiol from H2S-rich syngas over sulfided Mo-based catalysts", JOURNAL OF MOLECULAR CATALYSIS A: CHEMICAL, ELSEVIER, AMSTERDAM, NL, vol. 283, no. 1-2, 18 March 2008 (2008-03-18), pages 69-76, XP025469728, ISSN: 1381-1169, DOI: 10.1016/J.MOLCATA.2007.12.014 [retrieved on 2007-12-23]

**Beschreibung**

Die Erfindung betrifft ein Verfahren zur kontinuierlichen Herstellung von Methylmercaptan durch Umsetzen eines Gemisches, das kohlenstoffhaltige Verbindungen enthält, mit Schwefel und Wasserstoff, wobei die dabei entstehenden Verbindungen Schwefelkohlenstoff und Schwefelwasserstoff zu Methylmercaptan umgesetzt werden.

Methylmercaptan ist ein industriell wichtiges Zwischenprodukt für die Synthese von Methionin sowie für die Herstellung von Dimethylsulfoxid und Dimethylsulfon. Methylmercaptan wird überwiegend aus Methanol und Schwefelwasserstoff durch Reaktion an einem Katalysator, bestehend aus einem Aluminiumoxidträger und Übergangsmetalloxiden und basischen Promotoren hergestellt. Die Synthese des Mercaptans erfolgt gewöhnlich in der Gasphase bei Temperaturen zwischen 300 und 500°C und bei Drücken zwischen 1 und 25 bar. Das Produktgasgemisch enthält neben dem gebildeten Methylmercaptan und Wasser die nicht umgesetzte Anteile der Ausgangsstoffe Methanol und Schwefelwasserstoff und als Nebenprodukte Dimethylsulfid und Dimethylether, sowie in geringen Mengen auch Polysulfide (Dimethyldisulfid). Im Sinne der Reaktion inerte Gase wie beispielsweise Kohlenmonoxid, Kohlendioxid, Stickstoff und Wasserstoff sind auch im Produktgas enthalten.

Aus dem Produktgasgemisch wird das gebildete Methylmercaptan, wie z. B. in der US 5866721 erläutert, in mehreren Destillations- und Waschkolonnen bei Temperaturen zwischen 10 und 140°C abgetrennt.

Methylmercaptan kann alternativ aus Kohlenstoffoxiden, Wasserstoff, Schwefel und/oder Schwefelwasserstoff hergestellt werden. Gemäß US 4665242 erfolgt beispielsweise die Darstellung von Methylmercaptan über Katalysatoren auf der Basis von Alkalimetallwolframaten. Im Vergleich zum methanolbasierten Verfahren weisen diese Prozesse niedrigere Selektivitäten für Methylmercaptan und Umsätze an Kohlenstoffoxiden auf. Die US 4410731 betrifft ein Verfahren und Katalysatoren für die Darstellung von Methylmercaptan aus Kohlenstoffoxiden, Wasserstoff und Schwefelwasserstoff oder Schwefel auf der Basis von mit Übergangsmetalloxiden als Promotoren enthaltenden Alkalimetallmolybdänsulfiden und Aluminiumoxid als Träger. In der WO2005/040082 werden ein Verfahren und Katalysatoren für die Darstellung von Methylmercaptan aus Kohlenstoffoxiden, Wasserstoff und Schwefelwasserstoff oder Schwefel auf der Basis von Übergangsmetalloxide als Promotoren enthaltenden Alkalimetallmolybdaten beansprucht, bei denen vorzugsweise Siliziumdioxid als Träger dient.

Auch die internationale Offenlegungsschrift WO 2009/083368 A2 und der Artikel "Direct synthesis of methanethiol from $H_2S$-rich syngas over sulfided Mo-based catalysts" von Chen et al., Journal of Molecular Catalysis A: Chemical, Elsevier, Band 283, Nr. 1-2, 18. März 2008, Seiten 69-76, offenbaren Verfahren und Katalysatoren für die Darstellung von Methylmercaptan aus Kohlenstoffoxiden, Wasserstoff und Schwefelwasserstoff. Die mit diesen Verfahren erzielten Selektivitäten für die Bildung von Methylmercaptan sind jedoch vergleichsweise niedrige und machen diese Verfahren daher unattraktiv für eine technische Anwendung.

In der US 2008/0293974A1 werden ein Verfahren und Katalysatoren für die Darstellung von Methylmercaptan aus kohlenstoff- und wasserstoffhaltigen Verbindungen, Schwefel und oder Sauerstoff auf der Basis von mit Übergangsmetalloxiden promotierten Alkalimetallmolybdaten beansprucht.

Die Darstellung von Methylmercaptan aus Schwefelkohlenstoff oder Carbonylsulfid und Wasserstoff ist eine weitere Alternative zum Methanol basierten Verfahren. Die Prozesse sind jedoch charakterisiert durch vergleichsweise niedrige Selektivitäten für Methylmercaptan, eine Vielzahl von Nebenprodukten, die aufwendig und kostenintensiv abzutrennen sind, sowie die Notwendigkeit, toxischen Schwefelkohlenstoff bzw. Carbonylsulfid in großen Mengen handzuhaben.

In der US 4,057,613 wird ein katalysatorfreies Verfahren zur Herstellung von Schwefelkohlenstoff aus Schwefel und Kohlenwasserstoffen beschrieben.

Bell et al beschreiben in der US 2,565,195 ein Verfahren zur Hydrierung von Schwefelkohlenstoff zu Methylmercaptan und Dimethylsulfid über sauren Katalysatoren vom Friedel Crafts-Typ ($AlCl_3$, $BF_3$). Als Nebenprodukte fallen u.a. Schwefelwasserstoff, Thioformaldehyd, Trithiomethylen, Methylendithiol, Methan und Ethen an.

Audeh et al beschreiben in der US 4,822,938 die Umsetzung von Methan mit Schwefel zu Schwefelkohlenstoff, Methylmercaptan und höheren Kohlenwasserstoffen. Zum Einsatz kommen Katalysatoren auf Basis von Co-Ni-Systemen und H-ZSM-5-Zeolithen. Ziel der Reaktion ist der sukzessive Aufbau von höheren Aliphaten und Olefinen auf Basis von Methan in einer Fischer-Tropsch analogen Umsetzung. Schwefelkohlenstoff und Methylmercaptan werden hierbei als Intermediate bzw. Zwischenprodukte gesehen. Nachteil der Reaktion sind vergleichsweise hohe Reaktionstemperaturen von 580-640°C und ein breites Spektrum an schwefelhaltigen Reaktionsprodukten wie z. B. Schwefelwasserstoff

Gemäß US 4,864,074 von Han et al erfolgt die Umsetzung von Schwefel mit Methan zu Methylmercaptan und höheren Kohlenwasserstoffen über Aluminiumoxid und Zeolithen. Auch bei diesem Verfahren bildet sich Schwefelwasserstoff in stöchiometrischen Mengen.

Die direkte Umsetzung von bei anderen Prozessen anfallenden Gemischen, die Methan oder höhere, insbesondere gesättigte Kohlenwasserstoffe, gegebenenfalls Wasser, Wasserstoff und schwefelhaltige Verbindungen enthalten, insbesondere Schwefelkohlenstoff zu Methylmercaptan, gelingt bis dato nicht in technisch relevanten Ausbeuten und Selektivitäten, sondern führt zu einer Vielzahl von Nebenprodukten. Es sind teils aufwendige Reinigungsverfahren notwendig, bei denen eine Vielzahl von Nebenkomponenten nicht in den Prozess zurückgeführt werden können. Das verringert die Gesamtselektivität für Methylmercaptan und somit die Wirtschaftlichkeit des Verfahrens.

Die Umsetzung von Kohlenwasserstoffen mit Schwefel ist stets durch die Bildung von Schwefelwasserstoff begleitet. Dies stellt einen signifikanten Nachteil dar, da der anfallende Schwefelwasserstoff nach dem Stand der Technik nicht in einem anderen Prozess genutzt werden kann und, über Verbrennung mit Luft bzw. Sauerstoff im Rahmen einer Claus-analogen Reaktion zu elementarem Schwefel und Wasser umgesetzt werden muss. Dies mindert auf Grund eines zusätzlichen Investments für eine $H_2S$-Regenerationsanlage (Claus-Prozess) und den Verlust der Wertkomponente Wasserstoff bei der $H_2S$-Verbrennung die Gesamtwirtschaftlichkeit des Verfahrens.

Aufgabe der Erfindung ist die Bereitstellung eines wirtschaftlichen Verfahrens zur Herstellung von Methylmercaptan aus Mischungen, die insbesondere Kohlenwasserstoffe und Schwefel enthalten, wobei der anfallende Schwefelwasserstoff wirtschaftlich sinnvoll genutzt wird. Der dabei als Zwischenprodukt entstehende Schwefelkohlenstoff kann auch aus anderen Quellen stammen.

Gegenstand der Erfindung ist ein Verfahren zur Herstellung von Methylmercaptan, enthaltend die Schritte

a) Hydrierung von Schwefelkohlenstoff in Gegenwart eines Katalysators auf Basis von Alkalimolybdaten oder Alkaliwolframaten, die auf einem Träger aufgebracht sind, und

b) Umsetzung des bei dieser Reaktion gebildeten Schwefelwasserstoff enthaltenden Reaktionsgemisches mit mindestens einer der Verbindungen, ausgewählt aus der Gruppe Aldehyden, Ether, Alkohole, CO, $CO_2$, CO+$CO_2$ und COS in Gegenwart eines Metalloxidkatalysators auf Basis von Alkalimolybdaten oder Alkaliwolframaten, die auf einem Träger aufgebracht sind, wobei Wasserstoff nur nach Bedarf zugesetzt wird.

Die Umsetzung mit den Kohlenstoffoxiden erfolgt in Gegenwart von Wasserstoff. Die weiteren genannten sauerstoffhaltigen Verbindungen sind in diesem Fall bevorzugt als Co-Feed einzustzen.

Man kann dieses Verfahren bevorzugt an die Herstellung des Schwefelkohlenstoffs anschließen. Schwefelkohlenstoff kann explizit auch über die Disproportionierung von Carbonylsulfid (COS) zu Schwefelkohlenstoff ($CS_2$) und Kohlenstoffdioxid gewonnen werden.

Das bei der Herstellung von Schwefelkohlenstoff anfallende Reaktionsgemisch enthält zusätzlich zu dem bei der gegebenenfalls nicht umgesetzten Schwefel und Kohlenwasserstoff(en) Schwefelkohlenstoff, den gleichzeitig entstandenen Schwefelwasserstoff und gegebenenfalls weitere Verbindungen und wird als Eduktgemisch bezeichnet.

In einer bevorzugten Ausführungsform trennt man das bei der Hydrierung des Schwefelkohlenstoffs gebildete Methylmercaptan vor der Umsetzung des in dem dabei gebildeten Reaktionsgemisch enthaltenen Schwefelwasserstoffs zum Methylmercaptan ab.

Die Hydrierung des Schwefelkohlenstoffs findet bei einem Reaktionsüberdruck von mindestens 5 bar, insbesondere bis 50 bar und einer Temperatur von mindestens 200°C, insbesondere bis 350°C statt, wobei das molare $CS_2$/$H_2$/$H_2S$-Verhältnis im Bereich von 1:1:1 bis 1:10:10 liegt, bevorzugt im Bereich von 1:2:2 bis 1:5:5.

Der im Überschuss vorliegende Wasserstoff dient bei der folgenden Umsetzung von Schwefelwasserstoff mit CO und/oder $CO_2$ als Reaktionskomponente und muss dort nur noch nach Bedarf zugesetzt werden.

Die Wirtschaftlichkeit des Gesamtprozesses der Herstellung von Methylmercaptan, die über Schwefelkohlenstoff als Vorprodukt verläuft, hängt entscheidend von der Produktselektivität für Methylmercaptan, bezogen auf die eingesetzte Kohlenstoffquelle, ab. Durch den erfindungsgemäßen Einsatz von kohlenstoffhaltigen Verbindungen, die in anderen Prozessen als Nebenkomponenten von bzw. als Abfallströme anfallen oder bisher nur als Brennstoff zur Energieerzeugung genutzt werden, kann ein zusätzlicher Kostenvorteil erzielt werden. Beispiele für Quellen dieser Ausgangsgemische zur Herstellung von Schwefelkohlenstoff sind Erdgase, die schwefelhaltige Komponenten (z.B. $H_2S$, COS) als Verunreinigungen enthalten, und schwefelkohlenstoffhaltige Stoffströme. Die Gemische enthalten im Allgemeinen bevorzugt Methan und eine oder mehrere gesättigte oder ungesättigte Kohlenwasserstoffe mit $C_2$-$C_6$-Rest. Als weitere Quelle für Ausgangsmischungen dienen technische Verfahren zur Erzeugung von organischen Stickstoff- oder Schwefelverbindungen, in denen größere Mengen von Nebenprodukten anfallen, die aber in der Regel nach dem Stand der Technik ohne weitere Wertschöpfung verbrannt oder anderweitig entsorgt werden müssen. Insbesondere können in dem erfindungsgemäßen Verfahren in solchen Verfahren anfallende Abgasströme, die beispielsweise $H_2S$, COS, $SO_2$, $SO_3$-haltige Verbindungen, Alkylsulfide oder Alkylpolysulfide enthalten, eingesetzt werden. Hierzu zählen explizit auch Gase, die aus Abgasströmen von Anlagen zur Erzeugung von Energie oder chemischen Produkten direkt oder über Separationstechniken gewonnen werden, bzw. im Rahmen von biologischen Stoffwechselprozessen (z.B. Fermentations- und Abbauprozesse) entstehen. Diese Gasmischungen können als Hauptkomponenten Kohlenwasserstoffe, insbesondere Methan, Kohlenstoffoxide, organische Schwefel- und Stickstoffverbindungen oder Schwefelwasserstoff neben weiteren Verbindungen enthalten und werden bevorzugt in dem erfindungsgemäßen Verfahren verwendet.

Die direkte Umsetzung von Ausgangsgemischen, wie z. B. Erdgas mit der Hauptkomponente Methan, Kohlenwasserstoffe z.B. aus Schwerölfraktionen, Rückständen aus der Erdölraffination oder allgemein höheren Kohlenwasserstoffen, wie z.B. Oligomere, Polymere oder polycyclische Aromaten , die normalerweise z. B. auch als Abfallströme in anderen chemischen Prozessen anfallen, mit Schwefel und die anschließende Hydrierung des Schwefelkohlenstoffs zu Methylmercaptan unter weiterer Umsetzung des gleichzeitig gebildeten Schwefelwasserstoffs ist Gegenstand der Erfindung. Sie weist aufgrund deutlich niedrigerer Rohstoffkosten, einen signifikanten Kostenvorteil in den variablen Betriebskosten auf. Als Kohlenwasserstoffe werden eingesetzt, einzeln oder im Gemisch:

Alkane mit 1 bis 20 Kohlenstoffatomen, insbesondere 1 bis 8, bevorzug 1 bis 4 Kohlenstoffatome, insbesondere Methan als der Hauptbestandteil der Kohlenwasserstoffe, mit 30 bis 100 Vol-%., gesättigte cyclische oder ungesättigte cyclische Kohlenwasserstoffverbindungen, zu denen auch polycyclische Aromaten zählen.

Als Ether wird bevorzugt Diethylether, als Aldehyd Formaldehyd eingesetzt.

Zu den Alkoholen zählen Methanol, Ethanol, Propanol, Alkohole mit 1-10 Kohlenstoffatomen und mindestens einer Hydroxy-Gruppe, Polyole und insbesondere Methanol.

Die Erfindung zeichnet sich in einer Ausführungsform dadurch aus, dass Schwefelwasserstoff, der z.B. während der Methylmercaptanbildung aus Schwefelkohlenstoff ensteht, durch bevorzugt simultane Umsetzung mit Kohlenstoff und Sauerstoff enthaltenden Verbindungen zu Methylmercaptan umgesetzt wird und nicht durch einen nachgeschalteten Claus-Prozess zu Schwefel oder andersweitig aufgearbeitet werden muss.

Die kohlenstoffhaltigen Verbindungen bzw. Kohlenwasserstoffe können in festen, flüssigen oder gasförmigem Zustand bereitgestellt werden, liegen aber zum Zeitpunkt der Umsetzung bevorzugt gasförmig vor. Vorteilhafterweise wird als Kohlenwasserstoff liefernde Quelle Erdgas mit den Hauptbestandteilen Methan oder Ethan dem Prozess zur Herstellung von Schwefelkohlenstoff als Edukt zugeführt. Daneben kann das Eduktgas organische Schwefelverbindungen oder $H_2S$ enthalten. Ebenfalls enthalten sind im Allgemeinen inerte Gase, wie z. B. Stickstoff.

Die Umsetzung dieser Gasmischungen mit Schwefel erfolgt ohne weitere Aufarbeitung der Gase bei einem Reaktionsdruck von mindestens 5 bar, insbesondere 5 bis 50 und einer Temperatur von mindestens 200°C, insbesondere 300-600°C mit flüssigem oder gasförmigen Schwefel in einem ein- oder mehrstufigen Verfahrensschritt, gegebenenfalls in Gegenwart eines aus dem Stand der Technik bekannten Katalysators, z. B. Co-Ni-Systemen oder H-ZSM5-Zeolithen.

Schwefel wird dabei mit einem Überschuss von 1 bis 30% gegenüber der stöchiometrischen notwendigen Menge eingesetzt.

Schwefelkohlenstoff resultiert als Hauptprodukt dieser Umsetzung. Als Nebenprodukte fallen gegebenenfalls Sulfide, Polysulfide und Thiole an. Da das Eduktgemisch wasserstoffhaltige Komponenten enthält, wird stets das Koppelprodukt $H_2S$ gebildet.

Im gleichen oder optional einem weiteren Verfahrensschritt wird das so erhaltene Reaktionsgemisch mit Wasserstoff bei einem Reaktionsdruck von mindestens 5 bar, insbesondere 5 bis 50 bar und einer Temperatur von mindestens 200°C, insbesondere 250°C bis 450°C über einem Katalysator zu einer Reaktionsmischung umgesetzt, die als Hauptprodukt Methylmercaptan und weiteren Schwefelwasserstoff enthält.

Vor dessen Umsetzung erfolgt bevorzugt die Abtrennung des Methylmercaptans aus der Reaktionsmischung mit an sich bekannten Verfahren.

Schwefelkohlenstoff kann explizit auch über die Disproportionierung von Carbonylsulfid (COS) zu Schwefelkohlenstoff ($CS_2$) und Kohlenstoffdioxid gewonnen werden. Carbonylsulfid fällt u.a. bei der Umsetzung von Kohlenstoffoxiden (CO, $CO_2$) mit Schwefel an. Die Hydrierung von so gebildetem Schwefelkohlenstoff, welcher auch in Gemischen mit Carbonylsulfid vorliegen kann, erfolgt in analoger Weise, wie oben beschrieben.

Wie oben erläutert, ist das zu lösende Problem bei der Umsetzung von Kohlenwasserstoffen mit Schwefel und der Umsetzung von Schwefelkohlenstoff mit Wasserstoff, der Zwangsanfall des Koppelproduktes $H_2S$. Im erfindungsgemäßen Verfahren wird durch die Einspeisung von sauerstoffhaltigen Verbindungen, vorzugsweise Alkoholen, Ether oder Aldehyden, insbesondere Methanol, Dimethylether oder Formaldehyd, Carbonylsulfid oder Kohlenstoffoxiden (CO, $CO_2$, $CO+CO_2$) dieses $H_2S$ unter Bildung von Methylmercaptan stofflich genutzt, so dass ich die Gesamtausbeute an Methylmercaptan, bezogen auf den eingesetzten Kohlenstoff erhöht. Das Verfahren lässt sich hierdurch besonders wirtschaftlich betreiben, da eine kostenaufwendige Aufarbeitung des Schwefelwasserstoffs mit einer anschließenden Umsetzung zu elementarem Schwefel (z.B. in einem Claus-Reaktor) entfällt. Ferner kann hierdurch der Holdup an $H_2S$ in der Anlage signifikant reduziert werden, was einen deutlichen Sicherheitsvorteil darstellt.

Im Anschluss an die Umsetzung des Schwefelwasserstoffs werden nach der Abtrennung des Methylmercaptans die nicht umgesetzten Einsatzstoffe oder Zwischenprodukte wie z. B. Schwefelkohlenstoff in den Prozess zurückgeführt.

Die Gesamtselektivität für die Bildung des Methylmercaptans wird durch Recyclierung der kohlenstoff-, wasserstoff- und schwefelhaltigen Verbindungen in den Prozess erhöht.

Ein besonderer Vorteil der Erfindung ist, dass (Poly-) Sulfide mit Selektivitäten kleiner 1 % anfallen und bedingt durch die Recyclierung in das Verfahren z. B. der sich toxisch verhaltende Schwefelkohlenstoff nicht aufwendig separiert werden muss..

Das Produktgasgemisch des letzten Verfahrensschrittes zur Umsetzung des Schwefelwasserstoffs enthält neben dem gebildeten Methylmercaptan und Wasser, nicht umgesetzte Ausgangsstoffe, z.B. Methan und gegebenenfalls andere Kohlenwasserstoffe, Methanol, Wasserstoff, sowie in Spuren Kohlendioxid, Kohlenmonoxid und Nebenprodukte wie Carbonylsulfid, Dimethylsulfid und in geringen Mengen auch Polysulfide (Dimethyldisulfid) und Schwefelkohlenstoff. Im Sinne der Reaktion inerte Gase, wie beispielsweise Stickstoff sind auch im Produktgas enthalten.

Aus dem Produktgasgemisch wird das gebildete Methylmercaptan, z. B. wie in DE-A-1768826 erläutert, bevorzugt in mehreren Destillations- und Waschkolonnen bei Temperaturen zwischen 10 und 140°C abgetrennt. Kohlendioxid, Kohlenmonoxid, Wasserstoff, Schwefelwasserstoff und als Nebenprodukte Carbonylsulfid, Dimethylsulfid und in geringen Mengen auch Polysulfide (Dimethyldisulfid) und Schwefelkohlenstoff werden in den Prozess zurückgeführt. Vorteilhafterweise wird dieser Stoffstrom in einem optionalen Verfahrensschritt bevorzugt katalytisch mit Wasser in der Weise umgesetzt, dass das in den Prozess zurückgeführte Kreisgas nur noch eine oder mehrere des Hauptkomponenten Methanol, $CS_2$, $CO_2$, $CO$, $H_2$ und $H_2S$ enthält, welche unter den beschriebenen Prozessbedingungen in Methylmercaptan überführt werden können.

Die Wirtschaftlichkeit des Gesamtprozesses wird dadurch erhöht, dass vorteilhafterweise vor der Dosierung des Eduktgemisches in den Prozess keine aufwendige und kostenintensive Abtrennung von potentiellen Katalysatorgiften, wie z.B. schwefelhaltigen Verbindungen und elementarem Schwefel notwendig ist. Ebenso entfällt eine Abtrennung solcher Verbindungen nach der Umsetzung in der Verfahrensstufe zur Bildung von Schwefelkohlenstoff. Diese Stoffe können zusammen mit den zurückgeführten Reaktionsgasen ohne weitere Aufarbeitung und Kompression der Gase, direkt dem Prozess zugeführt werden, was einen signifikanten Kostenvorteil hinsichtlich der Investitions-und Betriebskosten des Prozesses darstellt. Vorteilhafterweise können Schwefel oder schwefelhaltige Schlacken, die gegebenenfalls als Nebenprodukte des Verfahrens anfallen, direkt in fester, flüssiger oder gasförmiger Form als Edukt dem Prozess wieder zugeführt werden. Hierzu zählen explizit auch Gase, die aus Abgasströmen von Anlagen zur Erzeugung von Energie oder chemischen Produkten direkt oder über Separationstechniken gewonnen werden, bzw. im Rahmen von biologischen Abbau- und Stoffwechselprozessen entstehen und direkt dem zweiten Verfahrensschritt zugeführt werden können. Diese Gasmischungen können als Hauptkomponenten Kohlenwasserstoffe, Alkohole, Kohlenstoffoxide, Schwefelund Stickstoffverbindungen in einer Gesamtkonzentration von 5 bis 90 Vol.-%, insbesondere 50 bis 90 Vol.-% neben anderen Stoffen enthalten.

Die Umsetzung der Gasmischung mit flüssigem oder gasförmigem Schwefel kann optional unter Verwendung eines Katalysators durch Reaktion in einem ein- oder mehrstufigen Verfahrensschritt erfolgen.

Für die Umsetzung von Schwefelkohlenstoff zu Methylmercaptan wird bevorzugt kein Vollumsatz an Wasserstoff angestrebt. Die Reaktion wird so durchgeführt, dass nach der Umsetzung das molare $CS_2$ / $H_2$ / $H_2S$-Verhältnis 1:1 :1 bis 1 : 10 : 10, insbesondere 1 :1:1 bis 1:5:10 beträgt. Vorteilhafterweise verlässt das Reaktionsgas aus der Umsetzung der Kohlenwasserstoffe mit Schwefel diesen Verfahrensschritt bei einem Druck von mindestens 5 bar, insbesondere 5 bis 50 bar und kann direkt ohne weitere Kompression der Hydrierung zugeführt werden. Dies stellt einen signifikanten Kostenvorteil dar, da auf eine Verdichterstufe mit hohen Investitions- und Betriebskosten verzichtet werden kann. Optional können Vorrichtungen zur Abtrennung von elementarem Schwefel oder schwefelhaltigen Nebenprodukten der Hydrierung vorgeschaltet sein.

Die sich anschließende Umsetzung des Schwefelwasserstoffs zu Methylmercaptan erfolgt in einem weiteren Verfahrensschritt über Katalysatoren. Es hat sich aber als eine vorteilhafte Variante erwiesen, den Schwefelwasserstoff zuvor aus dem in den vorliegenden Reaktionen entstandenen Reaktionsgemisch abzutrennen. Als vorteilhaft für die Umsetzung haben sich hierfür Metalloxidkatalysatoren erwiesen. Vorzugsweise werden Katalysatoren auf Basis von Alkalimolybdaten oder Alkaliwolframaten verwendet, die auf Träger aufgebracht sein können (US 5852219). Insbesondere geeignet sind Trägerkatalysatoren, die oxidische Mo- und K-Verbindungen enthalten, wobei Mo und K in einer Verbindung enthalten sein können, wie z. B. $K_2MoO_4$, und mindestens eine aktive oxidische Verbindung der allgemeinen Formel $A_xO_y$ enthalten. Dabei bedeutet A ein Element der Mangan-, Chrom- oder Eisengruppe, insbesondere Mn oder Re, und x und y ganze Zahlen von 1 bis 7. Der Katalysator enthält die Verbindungen bevorzugt in einem Gewichtsverhältnis von $A_xO_y$/$K_2MoO_4$/Träger = 0,001-0,5)/(0,01-0,8)/1 bzw. $A_xO_y$/$MoO_3$/$K_2O$/Träger [0,0001-0,5)/[0,01-0,8)/[0,005-0,5)/1 wobei die Gewichtsverhältnisse bevorzugt im Bereich

$$\mathtt{A_xO_y/K_2MoO_4/Tr\ddot{a}ger\ =\ (0,001\text{-}0,3)/(0,05\text{-}0,5)/1\ bzw.}$$

$$\mathtt{A_xO_y/MoO_3/K_2O/Tr\ddot{a}ger\ =\ [0,001\text{-}0,3)/(0,05\text{-}0,3)/0,03\text{-}0,3/1}$$

liegen.

Diese Katalysatoren enthalten bevorzugt einen oder mehrere Promotoren, ausgewählt aus der Gruppe der oxidischen Verbindungen mit der allgemeinen Formel $M_xO_y$, in denen M für ein Übergangselement oder ein Metall aus der Gruppe der Seltenen Erden steht und x und y eine ganze Zahl von 1 bis 7 bedeuten, entsprechend dem Oxidationsgrad der eingesetzten Elemente M.

Bevorzugt bedeutet M Fe, Co, Ni, La oder Ce. In einer besonderen Ausführungsform kann M auch Rb, Cs, Mg, Sr und Ba bedeuten. Die Verhältnisse der Gewichtsanteile liegen in den Bereichen:

$$K_2MoO_4/M_xO_y/Träger = (0,01-0,80(/(0,01-0,1)/1,$$

$$MoO_3/K_2O/M_xO_y/Träger = (0,10-0,50)/(0,10-0,30)/(0,01-0,1)/1,$$

Werden diese Katalysatoren vor dem Einsatz einer $H_2S$-haltigen Atmosphäre ausgesetzt, wandeln sich die oxidischen Metallverbindungen, mit denen nicht das Trägermaterial gemeint ist in sulfidische Verbindungen oder Mischungen aus oxidischen und sulfidischen Verbindungen um, die ebenso erfindungsgemäß einsetzbar sind.

Als Trägermaterialien werden bevorzugt Siliciumdioxide, Aluminiumoxide, Titandioxide, Zeolithe oder Aktivkohlen eingesetzt.

Titandioxid wird bevorzugt mit einem Gehalt von 60 Mol% Anatas eingesetzt.

Die Herstellung erfolgt in einem mehrstufigen Imprägnierverfahren, mit dem lösliche Verbindungen der gewünschten Promotoren oder aktiven oxidischen Verbindungen auf den Träger aufgebracht werden. Der imprägnierte Träger wird anschließend getrocknet und gegebenenfalls calciniert.

Vorzugsweise werden die Umsetzung der Kohlenwasserstoffe mit Schwefel und die Hydrierung des Schwefelkohlenstoffs zu Methylmercaptan in einem Reaktionsapparat kombiniert. Dies kann unter Verwendung von unterschiedlichen oder gleichen Katalysatoren geschehen. Vorteilhafterweise kommen Blasensäulen, Trickle-Bed-Reaktoren, Reaktivdestillationen, Festbett-, Horden- oder Rohrbündelreaktoren für die katalysierte Umsetzung zu Methylmercaptan zum Einsatz.

Die Umsetzung zu Methylmercaptan erfolgt bevorzugt über Katalysatoren auf Basis von Alkalimolybdaten oder Alkaliwolframaten. Bei einer Temperatur von 200 bis 600°C, vorzugsweise 250 bis 400°C und einem Druck von 1,5 bis 50 bar, vorzugsweise 8 bis 40 bar. Katalysatoren, die vorteilhafterweise zum Einsatz kommen, werden in den Anmeldungen WO 2005/040082, WO 2005/021491, WO 2006/015668 und WO 2006/063669 beschrieben.

In einer weiteren Ausführungsform der Erfindung werden die Umsetzung mit Schwefel und die Hydrierung zu Methylmercaptan in einer Vorrichtung kombiniert.

Die Auftrennung des Produktgasgemisches kann nach verschiedenen, bekannten Verfahren erfolgen. Eine besonders vorteilhafte Auftrennung wird in den Patentschriften EP-B- 0850923 (US 5866721) beschrieben.

Nicht umgesetzte Kohlenwasserstoffe, Alkohole, Kohlenstoffoxide, Schwefelkohlenstoff, Wasserstoff und Schwefelwasserstoff, sowie gasförmige Nebenprodukte, wie z.B. Carbonylsulfid werden in den Prozess zurückgeführt. Dies erfolgt in der Weise, dass die genannten Verbindungen vor der Methylmercaptanbildung aus Schwefelkohlenstoff eingespeist werden und dort in situ mit Schwefelwasserstoff, der als Koppelprodukt bei der Hydrierung von Schwefelkohlenstoff anfällt, zu Methylmercaptan umgesetzt werden. Ansonsten erfolgt die Rückführung durch Einspeisung in das Eduktgas. Vorteilhafterweise wird vor Rückführung in den Prozess das $CO_2$ (COS) / CO / $H_2$ / $H_2S$-Verhältnis durch Umsetzung mit Wasser auf 1 : 0,1 : 1 : 1 bis 1 : 1 : 10 : 10 eingestellt. Dies kann katalysiert oder nicht-katalysiert in einem Festbettreaktor, einem Reaktionsrohr, einer Waschkolonne oder einer Reaktivdestillation bei einer Temperatur von mindestens 120°C erfolgen.

Reaktionskomponenten, wie z.B. Sulfide, Polysulfide und Kohlenwasserstoffe, welche während der Abtrennung von Methylmercaptan im letzten Verfahrensschritt anfallen, können ohne weitere Aufarbeitung in den Prozess zurückgeführt werden, wodurch sich die Gesamtselektivität des Prozesses für Methylmercaptan bezogen auf Kohlenstoff auf über 95 % erhöht.

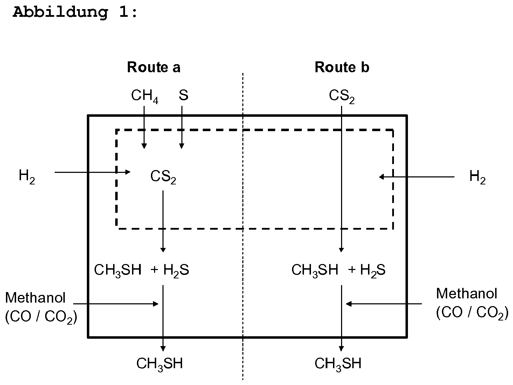

Die Abbildung 1 dient zur weiteren Erklärung des Verfahrens wobei "Route a" exemplarisch die Umsetzung von Methan mit Schwefel, Wasserstoff und Methanol oder CO / $CO_2$ zu Methylmercaptan und "Route b" die direkte Hydrierung von Schwefelkohlenstoff zu Methylmercaptan unter simultaner Umsetzung von Schwefelwasserstoff mit Methanol oder CO/$CO_2$ bezeichnet. Wichtig für die Wirtschaftlichkeit des Verfahrens ist die Möglichkeit, eine Vielzahl von festen, flüssigen und oder gasförmigen, kohlenstoff- und wasserstoffhaltigen Ausgangsstoffen zu verwenden, die in den Prozess mit Schwefel umgesetzt werden, und dass dieser Stoffstrom nicht aufwendig gereinigt und entschwefelt werden muss. Ferner können sämtliche Nebenprodukte, die nach der Umsetzung abgetrennt werden in den Prozeß recycliert werden. Vorteilhafterweise laufen, falls eine sequentielle Prozessführung gewählt wird, alle Reaktionen im gleichen Druckbereich ab, so dass auf eine kostenintensive Kompression der Gase zwischen den einzelnen Reaktionsschritten verzichtet werden kann. Die Umsetzungen erfolgen bei dem Ausgangsdruck der Gase, unter dem diese den ersten Verfahrensschritt verlassen. Vorteilhafterweise wird dieser Druck auf 5 bis 50 bar, insbesondere 8 bis 40 bar eingestellt. Im Sinne des Prozesses inerte Gase werden kontinuierlich oder diskontinuierlich über einen Purgegasstrom aus dem Prozess ausgeschleust.

Beispiele:

Der hier vorgestellte Prozess beinhaltet zwei Stufen.

In der ersten Stufe wurde Methan in flüssigem Schwefel bei einem Druck von 15-50bar und einer Temperatur von 500-550 °C zu Schwefelkohlenstoff und Schwefelwasserstoff umgesetzt. Dabei wurde das Methan durch die flüssige Schwefelphase geperlt und das Produktgas unmittelbar nach Verlassen der flüssigen Phase auf ca. 150 °C mittels eines auf den Reaktor gesetzten Kühlers abgekühlt.

Zum Reaktionsprodukt der ersten Stufe wurde Wasserstoff gegeben, mithilfe dessen der in der ersten Stufe gebildete Schwefelkohlenstoff in der zweiten Stufe bei 15-50bar und Temperaturen von 150 - 450°C zu Methylmercaptan hydriert wurde. Die Reaktanden wurden auf zwei Wegen bereitgestellt:

1. $H_2S$ und $CS_2$ wurden im Vorreaktor aus Methan und Schwefel hergestellt. (Beispiel 1)

2. $H_2S$ wurde im Vorreaktor aus $H_2$ und Schwefel hergestellt und $CS_2$ wurde über eine HPLC-Pumpe in den Vorreaktor gegeben. (Beispiel 2)

Die katalytische Aktivität wurde für einen Durchgang durch den Reaktor bestimmt (Single Pass).

**Beispiel 1**

Es wurde unter einem Druck von 15 bar Schwefel auf 500°C erhitzt und ein Gemisch aus Methan und Stickstoff (1:1) eingeleitet. Diese Prozedur führte zu einem Methanumsatz von 48.4 % unter stationären Bedingungen und somit zu einem Produktgasgemisch bestehend aus 16.3% $CS_2$, 17.4% $CH_4$, 33.7% $N_2$ und 32.6% $H_2S$. Kohlenstoffhaltige Nebenprodukte wurden nicht beobachtet (Selektivität zu $CS_2$ = 100%). Prozentangaben bei Gasgemischen sind als Vol.-% zu verstehen.

Diesem Produktgemisch wurde $H_2$ zugegeben und der zweiten Stufe zugeführt. Es ergab sich somit für die zweite Stufe eine Eduktzusammensetzung von 9% $CS_2$, 9.6% $CH_4$, 18.7% $N_2$, 18.2% $H_2S$ und 44.5% $H_2$. Für den Katalysator $K_2MoO_4/SiO_2$ sind die Umsätze, Ausbeuten und Selektivitäten dieser Hydrierung in Abhängigkeit der Temperatur in Tabelle 1 angegeben (Reaktionsdruck p = 15 bar).

Tabelle 1:

| Temp [°C] | Umsatz CS2 | Selektivität [%] | | | Ausbeute [%] | | |
|---|---|---|---|---|---|---|---|
| | | CH4 | MC | DMS | CH4 | MC | DMS |
| 250 | 17.16 | 0.00 | 93.37 | 0.26 | 0.00 | 16.02 | 0.04 |
| 300 | 66.97 | 0.00 | 90.18 | 0.29 | 0.00 | 60.39 | 0.19 |
| 325 | 95.65 | 0.00 | 83.15 | 0.33 | 0.00 | 79.53 | 0.31 |
| 350 | 99.97 | 0.00 | 83.07 | 0.40 | 0.00 | 83.04 | 0.40 |
| 375 | 99.97 | 3.23 | 79.12 | 0.39 | 3.23 | 79.09 | 0.39 |
| 400 | 99.97 | 10.09 | 71.57 | 0.45 | 10.09 | 71.55 | 0.45 |

Die für den Gesamtprozess bezogen auf das eingesetzte $CH_4$ resultierenden Produktselektivitäten und Ausbeuten sind in Tabelle 2 dargestellt.

Tabelle 2:

| Temp [°C] | Umsatz CH4 | Selektivität [%] | | | Ausbeute [%] | | |
|---|---|---|---|---|---|---|---|
| | | CS2 | MC | DMS | CS2 | MC | DMS |
| 250 | 49.67 | 79.30 | 15.34 | 0.09 | 39.39 | 7.62 | 0.04 |
| 300 | 49.67 | 31.62 | 57.81 | 0.37 | 15.71 | 28.72 | 0.18 |
| 325 | 49.67 | 4.16 | 76.14 | 0.60 | 2.07 | 37.82 | 0.30 |

(fortgesetzt)

| Temp [°C] | Umsatz CH4 | Selektivität [%] | | | Ausbeute [%] | | |
|---|---|---|---|---|---|---|---|
| | | CS2 | MC | DMS | CS2 | MC | DMS |
| 350 | 49.67 | 0.03 | 79.50 | 0.76 | 0.01 | 39.49 | 0.38 |
| 375 | 48.13 | 0.03 | 78.13 | 0.76 | 0.01 | 37.61 | 0.37 |
| 400 | 44.87 | 0.03 | 75.82 | 0.95 | 0.01 | 34.02 | 0.42 |

Beispiel 2

Es wurde nach der oben beschriebenen Option 2 verfahren (separate Einspeisung von Schwefelkohlenstoff). Die Bedingungen in der Vorstufe wurden so gewählt, dass vor der Hydrierstufe sich ein Gasgemisch aus 12.7% $N_2$, 9.9% $CS_2$, 12.6% $H_2S$ und 64.8% $H_2$ einstellte. Bei 20 bar und einem Gesamtfluss von 18.6 ml/min wurde für den Katalysator (28% $K_2MoO_4/SiO_2$) in Abhängigkeit der Hauptreaktortemperatur die folgenden Umsätze, Ausbeuten und Selektivitäten für die Hydrierung erzielt (Tabelle 3).

Tabelle 3:

| T [°C] | Umsatz CS2 [%] | Selektivität [%] | | | Ausbeute [%] | | |
|---|---|---|---|---|---|---|---|
| | | MC | DMS | CH4 | MC | DMS | CH4 |
| 150 | 1.14 | 100 | 0 | 0 | 1.14 | 0 | 0 |
| 165 | 0.03 | 100 | 0 | 0 | 0.03 | 0 | 0 |
| 180 | 0 | 100 | 0 | 0 | 0 | 0 | 0 |
| 195 | 0.8 | 100 | 0 | 0 | 0.8 | 0 | 0 |
| 210 | 6.84 | 100 | 0 | 0 | 6.84 | 0 | 0 |
| 225 | 10.39 | 100 | 0 | 0 | 10.39 | 0 | 0 |
| 240 | 26.32 | 100 | 0 | 0 | 26.32 | 0 | 0 |
| 255 | 53.91 | 98.02 | 1.5 | 0.48 | 52.84 | 0.81 | 0.26 |
| 270 | 90.07 | 97.8 | 1.13 | 1.07 | 88.08 | 1.02 | 0.96 |
| 285 | 99.94 | 97.92 | 0.92 | 1.15 | 97.87 | 0.92 | 1.15 |
| 300 | 100 | 97.54 | 0.87 | 1.6 | 97.54 | 0.87 | 1.6 |
| 315 | 100 | 96.7 | 0.87 | 2.42 | 96.7 | 0.87 | 2.42 |
| 330 | 100 | 94.96 | 0.83 | 4.22 | 94.96 | 0.83 | 4.22 |
| 345 | 100 | 92 | 0.85 | 7.15 | 92 | 0.85 | 7.15 |
| 360 | 100 | 87.34 | 1.09 | 11.58 | 87.34 | 1.09 | 11.58 |
| 375 | 100 | 79.81 | 1.24 | 18.95 | 79.81 | 1.24 | 18.95 |
| 390 | 100 | 69.24 | 1.44 | 29.32 | 69.24 | 1.44 | 29.32 |
| 400 | 100 | 61.27 | 1.53 | 37.19 | 61.27 | 1.53 | 37.19 |

Beispiel 3:

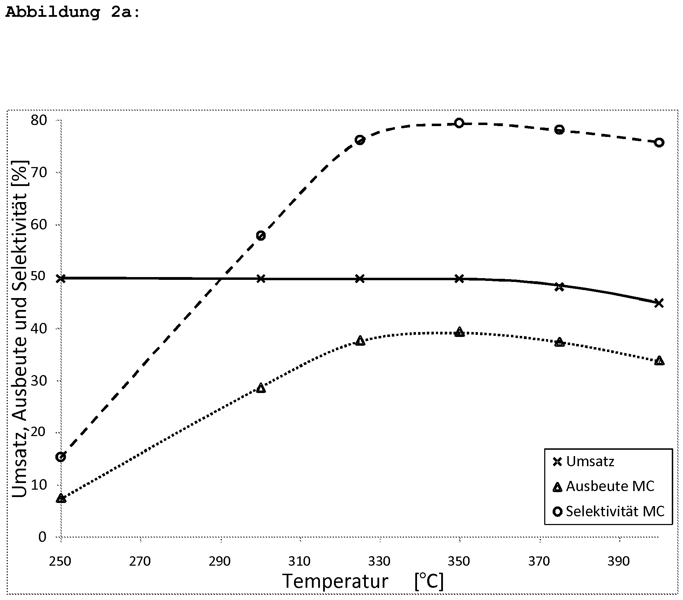

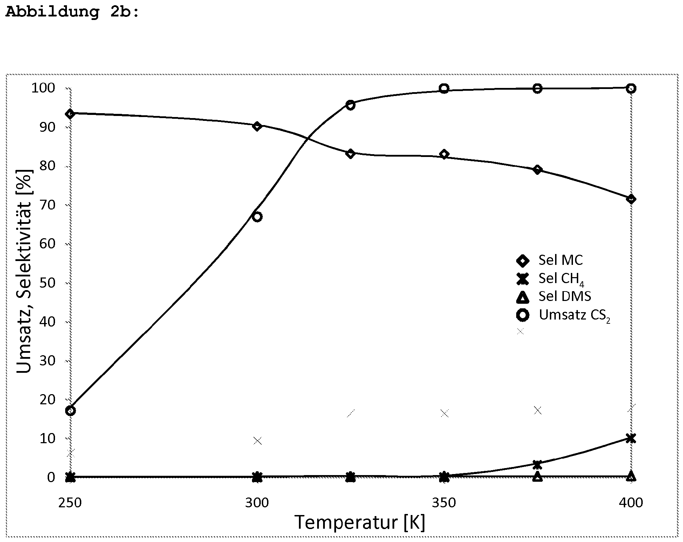

Abbildung 2a zeigt die Bildung von Schwefelkohlenstoff durch die Umsetzung von Schwefel mit Methan bei 525 °C mit anschließender Hydrierung zu Methylmercaptan über einem Katalysator der 2,9 m% CoO und 28m% $K_2MoO_4$ auf einem $SiO_2$-Träger enthält. Abbildung 2b zeigt die Produktselektivitäten und den $CS_2$-Umsatz für den der $CS_2$-Bildung folgenden Hydrierschritt.

**Beispiel 4:**

Bei der Bildung von Schwefelkohlenstoff aus Methan und Schwefel (Beispiel 1) fällt Schwefelwasserstoff als Koppelprodukt an ($CS_2$ : $H_2S$ = 1 : 2). Beim folgenden Hydrierschritt zu Methylmercaptan (siehe Beispiel 2) wird ebenfalls $H_2S$ als Koppelprodukt gebildet. Der resultierenden Produktgasmischung wurde bei einer Temperatur von 325°C und einem Reaktionsdruck von 15 bar (i) Methanol, (ii) CO oder (iii) $CO_2$ einzeln oder gemeinsam mindestens im Gesamt-Verhältnis 1,1 : 1 (MeOH + CO + CO2) : H2S) in Gegenwart des benötigten Wasserstoffs zugeführt. Für alle drei Reaktanden wurde ein $H_2S$-Umsatz > 95 % bei gleichzeitig erhöhten Methylmercaptan-Ausbeuten beobachtet. Durch Erhöhung des Methanol- / CO- oder $CO_2$-Anteils und Recyclierung von nicht umgesetzten Reaktanden (nach der Abtrennung von Methylmercaptan) konnte ein Vollumsatz an Schwefelwasserstoff erzielt werden.

**Patentansprüche**

1. Verfahren zur Herstellung von Methylmercaptan, enthaltend die Schritte

   a) Hydrierung von Schwefelkohlenstoff in Gegenwart eines Katalysators auf Basis von Alkalimolybdaten oder Alkaliwolframaten, die auf einem Träger aufgebracht sind, und
   b) Umsetzung des bei dieser Reaktion gebildeten Schwefelwasserstoff enthaltenden Reaktionsgemisches mit mindestens einer der Verbindungen, ausgewählt aus der Gruppe, bestehend aus Alkoholen, Ethern, Aldehyden, COS, CO, $CO_2$, CO+$CO_2$, in Gegenwart eines Metalloxidkatalysators auf Basis von Alkalimolybdaten oder Alkaliwolframaten, die auf einem Träger aufgebracht sind, wobei Wasserstoff nur nach Bedarf zugesetzt wird.

2. Verfahren gemäß Anspruch 1, bei dem das Schwefelkohlenstoff enthaltende Gemisch

   c) weitere Verbindungen, aus dem bei der Herstellung von Schwefelkohlenstoff aus Schwefel und Kohlenwasserstoffen anfallenden Reaktionsgemisch, oder
   d) weitere Verbindungen aus dem bei der Disproportionierung von Carbonylsulfid zu Schwefelkohlenstoff anfallenden Reaktionsgemisch, enthält.

3. Verfahren gemäß den Ansprüchen 1 oder 2, bei dem man das bei der Hydrierung des Schwefelkohlenstoffs gebildete Methylmercaptan vor der Umsetzung des in dem dabei gebildeten Reaktionsgemisch enthaltenen Schwefelwasserstoffs zum Methylmercaptan abtrennt.

4. Verfahren gemäß den Ansprüchen 1 und 2, bei dem man den Schwefelkohlenstoff bei einem Reaktionsdruck von mindestens 5 bar und einer Temperatur von mindestens 200°C umsetzt, wobei das molare $CS_2$/CO/$CO_2$/$H_2$/$H_2S$-Verhältnis im Bereich von 1:1:1:1:1 bis 1:10: 10:10:10 liegt.

5. Verfahren gemäß Anspruch 1, wobei der Schwefelwasserstoff mit Methanol umgesetzt wird.

6. Verfahren gemäß den Ansprüchen 1 und 2, bei dem das molare $CS_2$/$H_2$/$H_2S$-Verhältnis sich nach der Hydrierung des Schwefelkohlenstoffs auf 1:1:1 bis 1:10:10, insbesondere 1:1:1 bis 1:5:10 beläuft.

7. Verfahren gemäß Anspruch 2 wobei die Kohlenwasserstoffe oder kohlenstoffhaltigen Verbindungen aus Abgasströmen von Prozessen zur Erzeugung von Energie oder chemischen Produkten stammen.

8. Verfahren gemäß Anspruch 2, wobei die Kohlenwasserstoffe oder kohlenstoffhaltigen Verbindungen aus der Aufarbeitung von Prozessen zur Oxidation von Kohlenwasserstoffen und zur Synthese von Stickstoff-und schwefelhaltigen Verbindungen stammen.

9. Verfahren gemäß den Ansprüchen 1 bis 3, wobei die Kohlenwasserstoffe oder kohlenstoffhaltigen Verbindungen aus biologischen Stoffwechselprozessen stammen.

10. Verfahren gemäß den Ansprüchen 2-9, bei dem das Gemisch zur Herstellung von Schwefelkohlenstoff neben einer Verbindung, ausgewählt aus der Gruppe Methan, gesättigte oder ungesättigte Kohlenwasserstoffe mit $C_2$-$C_6$-Rest und Wasserstoff, mindestens eine der Verbindungen, ausgewählt aus der Gruppe, bestehend aus Alkoholen, Ether, Aldehyde, COS, CO, $CO_2$, CO+$CO_2$, enthält.

**11.** Verfahren gemäß den Ansprüchen 1 bis 9, bei dem der Schwefelkohlenstoff in Gegenwart von flüssigem oder gasförmigem Schwefel, in einer ein- oder mehrstufigen nichtkatalysierten Homogenreaktion oder unter Verwendung eines Katalysators gebildet wird.

**12.** Verfahren gemäß den Ansprüchen 1 bis 9, bei dem man nach Abtrennung des Methylmercaptans, nicht umgesetzte gasförmige Einsatzstoffe und Nebenprodukte abtrennt und in das Verfahren zurückführt.

**13.** Verfahren gemäß den Ansprüchen 1 bis 12, bei dem man die Gesamtmenge des Schwefelwasserstoffs durch Variation des Kohlenstoff-Wasserstoffverhältnises der im Eduktgemisch enthaltenen Verbindungen bzw. des $H_2$-Anteils im Reaktionsgas, das man dem Prozess zuführt und durch Variation eines oder mehrerer der Verfahrensparameter, ausgewählt aus der Gruppe: Verweilzeit, Reaktionstemperatur und Reaktionsdruck einstellt.

**14.** Verfahren gemäß den Ansprüchen 1 bis 9, bei dem man Reaktivdestillationen, Blasensäulen-, Festbett-, Trickle-Bed-, Horden- oder Rohrbündelreaktoren für die katalysierte Umsetzung zu Methylmercaptan einsetzt.

**15.** Verfahren gemäß den Ansprüchen 1 bis 9, bei dem man die Umsetzung der Kohlenwasserstoffe mit Schwefel und die Hydrierung des gebildeten Schwefelkohlenstoffs zu Methylmercaptan in einem Reaktionsapparat durchführt.

**16.** Verfahren gemäß den Ansprüchen 1 bis 15, bei dem das bei der Bildung von Schwefelkohlenstoff entstandene Reaktionsgemisch direkt ohne Druckverlust in einem zweiten Verfahrensschritt der Hydrierung des Schwefelkohlenstoffs zugeführt wird.

**17.** Verfahren gemäß den Ansprüchen 1 bis 16, bei dem man die Übergangsmetalloxide oder -sulfide als Promotoren enthaltende Alkaliwolframate oder Alkalimolybdate enthaltende Katalysatoren einsetzt.

**18.** Verfahren gemäß Anspruch 17, bei dem mindestens einer der Promotoren ausgewählt aus der Gruppe Chrom-, Eisen-, Cobalt-, Mangan- und Rhenium-Oxide oder Sulfide in den Alkaliwolframaten, Alkalimolybdaten oder halogenidhaltigen Alkaliwolframaten oder Alkalimolybdaten enthalten ist.

**19.** Verfahren gemäß den Ansprüchen 1, 17 oder 19, bei dem man Trägerkatalysatoren einsetzt, die oxidische Mo- und K-Verbindungen enthalten, wobei Mo und K in einer Verbindung enthalten sein können, und die mindestens eine aktive oxidische Verbindung der allgemeinen Formel $A_xO_y$ enthalten, A ein oder mehrere Elemente der Eisen- oder Mangangruppe, insbesondere Co, Mn oder Re, und x und y ganze Zahlen von 1 bis 7 bedeuten.

**Claims**

**1.** Process for preparing methyl mercaptan, comprising the steps of

a) hydrogenating carbon disulfide in the presence of a catalyst based on alkali metal molybdates or alkali metal tungstates applied to a support, and
b) reacting the reaction mixture comprising hydrogen sulfide formed in this reaction with at least one of the compounds selected from the group consisting of alcohols, ethers, aldehydes, COS, CO, $CO_2$, $CO+CO_2$, in the presence of a metal oxide catalyst based on alkali metal molybdates or alkali metal tungstates applied to a support, wherein hydrogen is only added if required.

**2.** Process according to Claim 1, in which the mixture comprising carbon disulfide comprises

c) further compounds from the reaction mixture obtained in the preparation of carbon disulfide from sulfur and hydrocarbons, or
d) further compounds from the reaction mixture obtained in the disproportionation of carbonyl sulfide to carbon disulfide.

**3.** Process according to Claim 1 or 2, in which the methyl mercaptan formed in the hydrogenation of the carbon disulfide is removed prior to the conversion of the hydrogen sulfide present in the reaction mixture thus formed to methyl mercaptan.

**4.** Process according to Claims 1 and 2, in which the carbon disulfide is converted at a reaction pressure of at least 5 bar and a temperature of at least 200°C, where the molar $CS_2/CO/CO_2/H_2/H_2S$ ratio is in the range from 1:1:1:1:1 to 1:10:10:10:10.

5. Process according to Claim 1, wherein the hydrogen sulfide is reacted with methanol.

6. Process according to Claims 1 and 2, in which the molar $CS_2/H_2/H_2S$ ratio after the hydrogenation of the carbon disulfide ranges from 1:1:1 to 1:10:10, especially 1:1:1 to 1:5:10.

7. Process according to Claim 2, wherein the hydrocarbons or carbon-containing compounds originate from offgas streams from processes for generating energy or chemical products.

8. Process according to Claim 2, wherein the hydrocarbons or carbon-containing compounds originate from the workup of processes for oxidizing hydrocarbons and for synthesizing nitrogen- and sulfur-containing compounds.

9. Process according to Claims 1 to 3, wherein the hydrocarbons or carbon-containing compounds originate from biological metabolism processes.

10. Process according to Claims 2-9, in which the mixture for preparing carbon disulfide, in addition to a compound selected from the group of methane, saturated or unsaturated hydrocarbons with a $C_2$-$C_6$ radical and hydrogen, comprises at least one of the compounds selected from the group consisting of alcohols, ethers, aldehydes, COS, CO, $CO_2$, $CO+CO_2$.

11. Process according to Claims 1 to 9, in which the carbon disulfide is formed in the presence of liquid or gaseous sulfur, in a one-stage or multistage non-catalyzed homogeneous reaction or using a catalyst.

12. Process according to Claims 1 to 9, in which, after removal of the methyl mercaptan, unconverted gaseous feedstocks and by-products are removed and recycled into the process.

13. Process according to Claims 1 to 12, in which the total amount of the hydrogen sulfide is adjusted by varying the carbon-hydrogen ratio of the compounds present in the reactant mixture or of the $H_2$ content in the reaction gas fed to the process, and by varying one or more of the process parameters selected from the group of: residence time, reaction temperature and reaction pressure.

14. Process according to Claims 1 to 9, in which reactive distillations, bubble column reactors, fixed bed reactors, trickle bed reactors, tray reactors or tube bundle reactors are used for the catalyzed conversion to methyl mercaptan.

15. Process according to Claims 1 to 9, in which the reaction of the hydrocarbons with sulfur and the hydrogenation of the carbon disulfide formed to methyl mercaptan are performed in one reaction apparatus.

16. Process according to Claims 1 to 15, in which the reaction mixture which arises in the formation of carbon disulfide is supplied directly with no pressure drop in a second process step to the hydrogenation of the carbon disulfide.

17. Process according to Claims 1 to 16, in which catalysts comprising alkali metal tungstates or alkali metal molybdates comprising the transition metal oxides or sulfides as promoters are used.

18. Process according to Claim 17, in which at least one of the promoters selected from the group of oxides or sulfides of chromium, of iron, of cobalt, of manganese and of rhenium is contained in the alkali metal tungstates, alkali metal molybdates or halogenated alkali metal tungstates or alkali metal molybdates.

19. Process according to Claim 1, 17 or 19, in which supported catalysts are used, which comprise oxidic molybdenum and potassium compounds, wherein Mo and K may be contained in one compound, and which comprise at least one active oxidic compound of the general formula $A_xO_y$, A is one or more elements from the iron or manganese group, especially Co, Mn or Re, and x and y are each integers from 1 to 7.

**Revendications**

1. Procédé de fabrication de méthylmercaptan, contenant les étapes suivantes :

a) l'hydrogénation de sulfure de carbone en présence d'un catalyseur à base de molybdates alcalins ou de tungstates alcalins, qui sont appliqués sur un support, et

b) la mise en réaction du mélange réactionnel contenant du sulfure d'hydrogène formé lors de cette réaction avec au moins un des composés choisis dans le groupe constitué par les alcools, les éthers, les aldéhydes, COS, CO, $CO_2$, CO+$CO_2$, en présence d'un catalyseur d'oxyde métallique à base de molybdates alcalins ou de tungstates alcalins, qui sont appliqués sur un support, de l'hydrogène n'étant ajouté qu'au besoin.

2. Procédé selon la revendication 1, dans lequel le mélange contenant du sulfure de carbone contient

c) d'autres composés, provenant du mélange réactionnel formé lors de la fabrication de sulfure de carbone à partir de soufre et d'hydrocarbures, ou

d) d'autres composés, provenant du mélange réactionnel formé lors de la dismutation de sulfure de carbonyle en sulfure de carbone.

3. Procédé selon la revendication 1 ou 2, dans lequel le méthylmercaptan formé lors de l'hydrogénation de sulfure de carbone est séparé avant la transformation du sulfure d'hydrogène contenu dans le mélange réactionnel formé en méthylmercaptan.

4. Procédé selon les revendications 1 et 2, dans lequel le sulfure de carbone est mis en réaction à une pression de réaction d'au moins 5 bar et à une température d'au moins 200 °C, le rapport molaire $CS_2$/CO/$CO_2$/$H_2$/$H_2S$ s'étend de 1:1:1:1:1 à 1:10:10:10:10.

5. Procédé selon la revendication 1, dans lequel le sulfure d'hydrogène est mis en réaction avec du méthanol.

6. Procédé selon les revendications 1 et 2, dans lequel le rapport molaire $CS_2$/$H_2$/$H_2S$ après l'hydrogénation du sulfure de carbone est de 1:1:1 à 1:10:10, notamment de 1:1:1 à 1:5:10.

7. Procédé selon la revendication 2, dans lequel les hydrocarbures ou les composés contenant du carbone proviennent de courants de gaz d'échappement de procédés pour la génération d'énergie ou de produits chimiques.

8. Procédé selon la revendication 2, dans lequel les hydrocarbures ou les composés contenant du carbone proviennent du traitement de procédés pour l'oxydation d'hydrocarbures et pour la synthèse de composés contenant de l'azote et du soufre.

9. Procédé selon les revendications 1 à 3, dans lequel les hydrocarbures ou les composés contenant du carbone proviennent de processus métaboliques biologiques.

10. Procédé selon les revendications 2 à 9, dans lequel le mélange pour la fabrication de sulfure de carbone contient en plus d'un composé, choisi dans le groupe constitué par le méthane, les hydrocarbures saturés ou insaturés contenant un radical en $C_2$-$C_6$ et de l'hydrogène, en outre au moins un des composés choisis dans le groupe constitué par les alcools, les éthers, les aldéhydes, COS, CO, $CO_2$, CO+$CO_2$.

11. Procédé selon les revendications 1 à 9, dans lequel le sulfure de carbone est formé en présence de soufre liquide ou gazeux, par une réaction homogène non catalysée en une ou plusieurs étapes ou utilisant un catalyseur.

12. Procédé selon les revendications 1 à 9, dans lequel, après la séparation du méthylmercaptan, les matières premières gazeuses non réagies et les produits secondaires sont séparés et recyclés dans le procédé.

13. Procédé selon les revendications 1 à 12, dans lequel la quantité totale du sulfure d'hydrogène est ajustée par variation du rapport carbone-hydrogène des composés contenus dans le mélange de réactifs ou de la proportion d'$H_2$ dans le gaz réactionnel, qui est introduit dans le procédé, et par variation d'un ou plusieurs paramètres du procédé choisis dans le groupe constitué par : le temps de séjour, la température de réaction et la pression de réaction.

14. Procédé selon les revendications 1 à 9, dans lequel des distillations réactives, des réacteurs à colonnes à bulles, à lit fixe, à lit ruisselant, à étages ou à faisceau de tubes sont utilisés pour la réaction catalysée en méthylmercaptan.

15. Procédé selon les revendications 1 à 9, dans lequel la réaction des hydrocarbures avec le soufre et l'hydrogénation du sulfure de carbone formé en méthylmercaptan sont réalisées dans un appareil de réaction.

16. Procédé selon les revendications 1 à 15, dans lequel le mélange réactionnel formé lors de la formation de sulfure de carbone est introduit directement sans perte de pression dans une seconde étape de procédé à l'hydrogénation du sulfure de carbone.

17. Procédé selon les revendications 1 à 16, dans lequel les oxydes ou sulfures de métaux de transition sont utilisés comme catalyseurs contenant des tungstates alcalins ou des molybdates alcalins contenant des promoteurs.

18. Procédé selon la revendication 17, dans lequel au moins un des promoteurs est choisi dans le groupe constitué par les oxydes ou sulfures de chrome, de fer, de cobalt, de manganèse et de rhénium dans les tungstates alcalins, les molybdates alcalins ou les tungstates alcalins ou les molybdates alcalins contenant des halogénures.

19. Procédé selon les revendications 1, 17 ou 19, dans lequel des catalyseur supportés sont utilisés, qui contiennent des composés de Mo et K oxydiques, Mo et K pouvant être contenus dans un composé, et qui contiennent au moins un composé oxydique actif de formule générale $A_xO_y$, A signifiant un ou plusieurs éléments du groupe du fer ou du manganèse, notamment Co, Mn ou Re, et x et y signifiant des nombres entiers de 1 à 7.

**Abbildung 1:**

Abbildung 2a:

Abbildung 2b:

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5866721 A
- US 4665242 A
- US 4410731 A
- WO 2005040082 A
- WO 2009083368 A2
- US 20080293974 A1
- US 4057613 A
- US 2565195 A
- US 4822938 A
- US 4864074 A
- DE 1768826 A
- US 5852219 A
- WO 2005021491 A
- WO 2006015668 A
- WO 2006063669 A

**In der Beschreibung aufgeführte Nicht-Patentliteratur**

- **CHEN et al.** Journal of Molecular Catalysis A: Chemical. Elsevier, 18. Marz 2008, vol. 283, 69-76